# EUROPEAN PATENT APPLICATION

(11) **EP 4 089 107 A1**
(43) Date of publication of application: **16.11.2022**
(21) Application number: 21173310.0
(22) Date of filing: 11.05.2021
(51) Int. Cl.: C07K 14/54, C07K 14/715

(54) **THERAPEUTIC DERIVATIVES OF INTERLEUKIN-22**

(71) Applicant: Cytoki Pharma ApS, 3500 Vaerløse (DK)
(72) Inventor: Sass-Oerum, Kristian, 1739 Copenhagen (DK)
(74) Representative: Cooley (UK) LLP

(57) **Abstract**

The invention relates to novel derivatives of Interleukin-22 (IL-22), particularly those comprising a fatty acid covalently attached to an IL-22 protein, wherein the IL-22 protein comprises at least one amino acid substitution, and their use in therapy.

## Description

### FIELD OF THE INVENTION

The present invention relates to novel derivatives of Interleukin-22 (IL-22), and in particular to derivatives comprising a fatty acid covalently attached to an IL-22 protein. The IL-22 protein is a variant of native mature human IL-22 (hereinafter "hIL-22") and the fatty acid is covalently attached to a particular substitution. The invention also encompasses methods for their production and their use in therapy, including the treatment, prevention and amelioration of metabolic, liver, pulmonary, gut, kidney, central nervous system (CNS) and skin diseases, disorders and conditions.

### BACKGROUND OF THE INVENTION

IL-22 is a 146 amino acid protein with a molecular weight of 17 KDa. It belongs to the IL-10 family of cytokines and selectively activates a heterodimeric receptor consisting of an IL-10 receptor B subunit (IL-10RA2), which is ubiquitously expressed, and an IL-22 receptor A subunit (IL-22RA1), which has an epithelial restricted expression. It is a unique cytokine in that it is released from immune cells, but selectively targets epithelial cells. Hence, the signaling pathways induced by IL-22 may have relevance in different tissues (targets include skin, intestine, lung, liver, kidney, pancreas and thymus), but IL-22 activates them in an epithelial-specific manner. A soluble binding protein, IL-22BP, neutralises IL-22 and thus regulates its effect.

IL-22 is released as a response to signals reflecting chemical or mechanical injury, e.g. aryl hydrocarbon receptor activation in response to environmental toxins or tryptophan intermediates, and the activation of pattern recognition receptors, such as toll-like receptor 4, in response to proteins, fragments and debris from dying cells or invading pathogens. IL-22 release is further stimulated by certain cytokines, in particular IL-23 and to a lesser extent IL1β. IL-22 is thus secreted as a response to cues reflecting pathogen infection and immune activation too.

The effect of IL-22 is the result of an orchestrated engagement of several activities/pathways. IL-22 acts on epithelial barrier tissues and organs upon injury to protect the cells and maintain barrier function (e.g. through activation of anti-apoptotic gene programs). It also accelerates repair (e.g. by inducing the proliferation of mature cells and activation of stem cells), prevents fibrosis (e.g. through reducing epithelial-mesenchymal transition, antagonising the NLRP3 inflammasome and inducing hepatic stellate cell senescence) and controls inflammation (e.g. by inducing anti-microbial peptides and chemotaxis signals). IL-22 has been reported as able to treat a range of medical conditions, including those often observed in diabetic or overweight mammals, such as hyperglycemia, hyperlipidemia and hyperinsulinemia.

However, IL-22 is generally cleared quickly from the body by the kidneys, which limits its use in clinical practice. Known methods for extending the half-life of circulating IL-22 therefore seek to artificially increase the size of IL-22 beyond 70 kDa, so as to avoid renal clearance. Ligating IL-22 to an Fc antibody fragment is currently the best solution to this effect; Genentech and Generon Shanghai both have long-acting IL-22-Fc fusions in clinical development. Modifying IL-22 with polyethylene glycol (PEGylation) is another known means for avoiding renal clearance.

However, these existing solutions are not without their disadvantages. The available data suggest that PEG itself is immunogenic and PEG-containing vacuoles are observed in cells with PEGylated biologicals. Decreased activity and heterogeneity are also disadvantageous aspects of PEGylation. Although Fc fusion technology is very well known, adding an Fc antibody fragment represents a major change in the structure of IL-22, which affects its properties beyond half-life extension. As Fc fusion increases the size of the protein from approximately 17 kDa to approximately 85 kDa, properties such as diffusion rate, distribution and receptor engagement kinetics may be affected. For example, some Fc fusions are slowly absorbed and/or are too large for administration via certain routes. Both Genentech and Generon also report moderate and reversible skin reactions as dose-limiting adverse effects of IL-22-Fc fusions. Furthermore, the potency may be affected through steric hindrance caused by the large fusion partner.

In addition, while the native IL-22 has a very short half-life of a few hours, which would strongly limit its clinical utility, the IL-22-Fc fusions have been reported to have a half-life of a week or longer in man. For some conditions it will be beneficial to have potent molecules with prolonged half-life compared to native IL-22, but a shorter half-life than the Fc-fusions, e.g. for optimal titration, local application and action. There are currently no solutions described for this problem.

A need therefore remains in the art for new biocompatible modifiers of IL-22 that enhance circulating half-lives, and half-life in local tissue compartments and biological fluids, and demonstrate optimised pharmacokinetic and pharmacodynamic properties compared to the native molecule. Ideally they should maintain potency and other properties of the native molecule and also avoid toxicity, immunogenicity and any other adverse reactions demonstrated by known derivatives.

### SUMMARY OF THE INVENTION

In a first aspect, there is provided a derivative of IL-22 comprising a fatty acid covalently attached to an IL-22 protein, wherein the IL-22 protein is a variant of hIL-22, said variant comprising a substitution at position 95 or 106 of hIL-22, and the fatty acid is covalently attached at said substituted position.

The substitution may be selected from R95C and L106C.

In embodiments of the invention, the fatty acid is covalently attached to the IL-22 protein by a linker.

The fatty acid may be of Formula I:

HOOC-(CH₂)ₓ-CO-^{∗},

wherein x is an integer in the range of 10-18, optionally 12-18, 14-16 or 16-18, and ^{∗} designates a point of attachment to the IL-22 protein or linker. It may be a fatty diacid, such as a C12, C14, C16, C18 or C20 diacid. Advantageously, the fatty acid is a C16 or C18 diacid, and most advantageously it is a C18 diacid.

The fatty monoacid may be of Formula II:

H₃C-(CH₂)ₓ-CO-*,

wherein x is an integer in the range of 10-18, optionally 12-18, 14-16 or 16-18, and ^{∗} designates a point of attachment to the IL-22 protein or linker. It may be a fatty monoacid, such as a C12, C14, C16, C18 or C20 monoacid. Advantageously, the fatty acid is a C16 or C18 monoacid, and most advantageously it is a C16 monoacid.

The variant is a substituted form of hIL-22, comprising a substitution at position 95 or 106 of hIL-22. It may be substituted at one or more further positions, optionally position 1, 21, 35, 64, 113 and/or 114. It may comprise a substitution of hIL-22 selected from the group consisting of A1C, A1G, A1H, N21C, N21D, N21Q, N35C, N35D, N35H, N35Q, N64C, N64D, N64Q, N64W, R95C, L106C, Q113C, Q113R, K114C and K114R. Advantageously, the variant comprises a Gln residue at position 35 and 64 of hIL-22.

The variant may be an extended form of hIL-22. It may comprise an N-terminal peptide, such as an N-terminal trimer. Advantageously, the variant comprises an N-terminal G-P-G.

The linker may comprise one or more amino acids, optionally including glutamic acid (Glu) and/or lysine (Lys). The linker may include an oxyethylene glycine unit or multiple linked oxyethylene glycine units, optionally 2-5 such units, advantageously 2 units. The linker may comprise one or more oligo(ethylene glycol) (OEG) residues. It may comprise an ethylenediamine (C₂DA) group and/or an acetamide (Ac) group. Advantageously, the linker comprises all of the aforementioned elements in combination. In particular, the linker may be γGlu-OEG-OEG-C2DA-Ac, γGlu-γGlu-γGlu-γGlu-OEG-OEG-εLys-αAc or yGlu-OEG-OEG-εLys-αAc.

The linker may be a Cys-reactive linker attached to a Cys residue substituted at position 95 or 106 of hIL-22.

In an embodiment, the derivative comprises a C18 diacid covalently attached by a linker to the variant of hIL-22. Advantageously, the variant comprises a Cys residue substituted at position 95 or 106 of hIL-22 and the linker is attached to said Cys residue. Exemplary derivatives of the invention are those identified herein as Derivatives 1 and 2.

In a second aspect, there is provided a process for preparing a derivative of the first aspect comprising covalently attaching a fatty acid to an IL-22 protein, wherein the IL-22 protein is a variant of hIL-22, said variant comprising a substitution at position 95 or 106 of hIL-22, and the process comprises covalently attaching the fatty acid at said substituted position.

In a third aspect, there is provided a pharmaceutical composition comprising a derivative of the first aspect, and a pharmaceutically acceptable vehicle.

In a fourth aspect, there is provided a derivative of the first aspect or a pharmaceutical composition of the third aspect, for use in therapy.

In a fifth aspect, there is provided a derivative of the first aspect or a pharmaceutical composition of the third aspect, for use in a method of treating a metabolic, liver, pulmonary, gut, kidney, CNS or skin disease, disorder or condition.

The metabolic disease, disorder or condition may be obesity, diabetes type 1, diabetes type 2, hyperlipidemia, hyperglycemia or hyperinsulinemia.

The liver disease, disorder or condition may be non-alcoholic fatty liver disease (NAFLD), non-alcoholic steatohepatitis (NASH), cirrhosis, alcoholic hepatitis, acute liver failure, chronic liver failure, acute-on-chronic liver failure (ACLF), acute liver injury, acetaminophen induced liver toxicity, sclerosing cholangitis, biliary cirrhosis or a pathological condition caused by surgery or transplantation.

The pulmonary disease, disorder or condition may be chronic obstructive pulmonary disease (COPD), cystic fibrosis, bronchiectasis, idiopathic pulmonary fibrosis, acute respiratory distress syndrome, a chemical injury, a viral infection, a bacterial infection or a fungal infection.

The gut disease, disorder or condition may be inflammatory bowel disease (IBD), ulcerative colitis, Crohn's disease, graft-versus-host-disease (GvHD), a chemical injury, a viral infection or a bacterial infection.

The kidney disease, disorder or condition may be acute kidney disease or chronic kidney disease.

The CNS disease, disorder or condition may be multiple sclerosis.

The skin disease, disorder or condition may be a wound, inflammatory disease or GvHD

### BRIEF DESCRIPTION OF THE DRAWINGS

Figure 1 illustrates a (A) C18 diacid, (B) C16 diacid, and (C) C14 diacid, each connected to a linker comprising a Cys-reactive unit. These combinations of fatty acids and linkers may be employed in the derivatives of the invention. The combination of fatty acid and linker shown in (A) is employed in the derivatives identified herein as Derivatives 1 and 2.
Figure 2 illustrates the structure of a derivative of the invention identified herein as Derivative 1.
Figure 3 illustrates the structure of a derivative of the invention identified herein as Derivative 2.

### DETAILED DESCRIPTION

In what follows, Greek letters are represented by their symbol rather than their written name. For example, α = alpha, ε = epsilon, γ = gamma and µ = mu. Amino acid residues may be identified by their full name, three-letter code or one-letter code, all of which are fully equivalent.

The term "derivative of IL-22", as used herein, refers to an IL-22 protein having a covalently attached fatty acid, wherein the IL-22 protein is a variant of hIL-22, said variant comprising a substitution at position 95 or 106 of hIL-22, and the fatty acid is covalently attached at said substituted position. The term encompasses both derivatives in which the fatty acid is covalently attached to the IL-22 protein directly and those in which the covalent attachment is by a linker.

The covalent attachment of fatty acids is a proven technology for half-life extension of peptides and proteins and is a way of subtending a fatty acid from the peptide or protein. It is known from marketed products for types 1 and 2 diabetes, such as insulins Levemir^{®} (detemir) and Tresiba^{®} (degludec), and glucagon-like peptide-1 (GLP-1) derivatives Victoza^{®} (liraglutide) and Ozempic^{®} (semaglutide).

Fatty acid attachment enables binding to albumin, thereby preventing renal excretion and providing some steric protection against proteolysis. Advantageously, it offers a minimal modification to IL-22 compared to Fc fusion or PEGylation. In this regard, whilst Fc fusion and PEGylation aim to increase the size of IL-22 beyond the threshold for renal clearance, derivatives comprising a fatty acid covalently attached to an IL-22 protein retain a small size similar to that of the IL-22 protein. Thus, as the fatty acid attachment is a minimal modification, the resultant derivative is believed to maintain native-like properties including distribution, diffusion rate and receptor engagement (binding, activation and trafficking) and minimise immunogenicity risk.

As above, fatty acid attachment has proven therapeutic efficacy in insulin and GLP-1 derivatives for diabetes. However, IL-22 is a very different protein in terms of its size, sequence and biological properties. It was therefore counterintuitive to the inventors that fatty acids could be covalently attached to IL-22 whilst maintaining therapeutic effect. It was particularly surprising that such a minimal modification to IL-22 could result in high potency (identical or close to hIL-22) combined with a prolonged circulatory half-life or prolonged half-life in biological fluids (e.g. plasma or intestinal fluid) or tissue preparations.

In a first aspect, therefore, the invention relates to a derivative of IL-22 comprising a fatty acid covalently attached to an IL-22 protein, wherein the IL-22 protein is a variant of hIL-22, said variant comprising a substitution at position 95 or 106 of hIL-22, and the fatty acid is covalently attached at said substituted position. The fatty acid may be covalently attached to the IL-22 protein directly or via a linker, which itself can be devised of various subunits. The term, "IL-22 protein", as used herein, can mean a native IL-22 protein, such as hIL-22, or a variant thereof. A "variant" can be a protein having a similar amino acid sequence to that of the native protein, as further defined herein. The IL-22 protein included in the derivatives of the invention is a variant of hIL-22, said variant comprising a substitution at position 95 or 106 of hIL-22.

In nature, human IL-22 protein is synthesised with a signal peptide of 33 amino acids for secretion. The mature human IL-22 protein (i.e. hIL-22) is 146 amino acids in length and has 80.8% sequence identity with murine IL-22 (the latter being 147 amino acids in length). The amino acid sequence of hIL-22 is identified herein as SEQ ID NO. 1. Like other IL-10 family members, the IL-22 structure contains six α-helices (referred to as helices A to F).

The derivatives of the invention have one or more amino acid sequence variations within the native sequence of hIL-22. Specifically, they have an amino acid substitution at position 95 and/or 106. They may additionally include one or more amino acid sequence variations relative to (i.e. outside) the native sequence.

Expressions such as "within", "relative to", "corresponding to" and "equivalent to" are used herein to characterise the site of change and/or covalent attachment of a fatty acid in an IL-22 protein by reference to the sequence of the native protein, e.g. hIL-22. In SEQ ID NO. 1, the first amino acid residue of hIL-22 (alanine (Ala)) is assigned position 1.

Thus, a variation within the sequence of hIL-22 is a variation to any of residue numbers 1-146 in SEQ ID NO. 1. For example, a Glu substitution for the native Asp at residue 10 in hIL-22 is represented herein as "D10E". If the derivative also has a fatty acid covalently attached at position 10, it is herein referred to as attachment at residue "10E".

A variation relative to the sequence of hIL-22, however, is a variation external to residue numbers 1-146 in SEQ ID NO. 1. For example, a derivative as defined herein may include an N-terminal peptide of 15 amino acids in length. The residues in the N-terminal peptide are numbered negatively, starting from the residue attached to residue 1 in hIL-22, i.e. the first residue in the N-terminal peptide that is attached to residue 1 in hIL-22 is denoted "-1". Thus, if the derivative had a fatty acid covalently attached at the 7^{th} residue in the N-terminal peptide starting from position -1 and this were Cys, the covalent attachment site for the derivative would be herein referred to as "-7C". Naturally, however, the numbering used in the sequence listing for such a derivative would start from 1, in accordance with WIPO Standard ST.25; as such, position 1 in the sequence listing for the derivative would actually be residue -7 as referred to herein.

Two, three, four, five or more variations may be made within the native sequence to form the derivatives of the invention. For example, more than 10, 15, 20, 25, 50, 75, 100 or even more than 125 variations may be made in this regard. Any of residues 1-146 in the native sequence may be varied. Exemplary residues for variation are residues 1, 2, 3, 4, 5, 6, 7, 8, 9, 10, 11, 12, 13, 14, 15, 16, 17, 18, 19, 20, 21, 22, 24, 25, 26, 27, 29, 30, 32, 33, 34, 35, 36, 37, 38, 39, 40, 41, 42, 44, 45, 47, 48, 49, 50, 51, 52, 53, 54, 55, 56, 58, 59, 61, 62, 63, 64, 65, 67, 68, 69, 70, 71, 72, 73, 74, 75, 77, 78, 79, 82, 83, 84, 86, 88, 90, 91, 92, 93, 94, 95, 96, 97, 98, 99, 100, 102, 103, 104, 105, 106, 107, 108, 109, 110, 111, 112, 113, 114, 115, 116, 117, 118, 119, 120, 121, 122, 123, 124, 126, 127, 128, 129, 130, 132, 133, 134, 135, 137, 138, 139, 141, 143, 144, 145 and/or 146 in hIL-22. Variation at residues 1, 21, 35, 64, 95, 106, 113 and/or 114 is particularly advantageous. At the very least, the derivatives of the invention include variation at residue 95 or 106.

The variations within the native sequence are typically amino acid substitutions. The term "substitution", as used herein, can mean the replacement of an amino acid in the native protein with another. They may be conservative or non-conservative substitutions. Substitutions at position 95 or 106 of hIL-22 are necessarily employed in the invention. Advantageously the substitution is R95C or L106C. Additional, exemplary substitutions are A1C, A1G, A1H, P2C, P2H, I3C, I3H, I3V, S4H, S4N, S5H, S5T, H6C, H6R, C7G, R8G, R8K, L9S, D10E, D10S, K11C, K11G, K11V, S12C, N13C, N13G, F14S, Q15C, Q15E, Q16V, P17L, Y18F, I19Q, T20V, N21C, N21D, N21Q, R22S, F24H, M25E, M25L, L26S, A27L, E29P, A30Q, L32C, L32R, A33C, A33N, D34F, N35C, N35D, N35H, N35Q, N36Q, T37C, T37I, D38L, V39Q, R40W, L41Q, I42P, E44R, K45A, F47T, H48G, H48R, G49N, V50S, S51C, M52A, M52C, M52L, M52V, S53C, S53K, S53Y, E54D, E54F, R55Q, R55V, C56Q, L58K, M59I, Q61E, V62D, L63C, N64C, N64D, N64Q, N64W, F65G, L67Q, E69D, E69L, V70S, L71C, F72D, F72L, P73C, P73L, Q74T, R77I, F78Q, Q79E, M82Y, Q83G, E84R, V86A, F88N, A90P, A90T, R91C, R91K, R91Y, L92R, S93Y, N94C, N94Q, R95K (if R95C is not already employed), R95Q (if R95C is not already employed), L96E, S97K, T98C, T98N, T98S, C99V, H100S, E102S, G103D, D104Y, D105Y, L106E (if L106C is not already employed), L106Q (if L106C is not already employed), H107L, H107N, I108L, Q109Y, R110C, R110K, N111K, V112E, Q113C, Q113R, K114C, K114R, L115V, K116Y, D117E, T118G, V119A, K120H, K121R, L122A, G123V, G126Y, E127C, I128V, K129V, G132Y, E133Q, L134P, D135M, L137D, F138R, M139L, M139R, L141Q, N143S, A144E, C145E, I146R and/or I146V. Advantageously, the additional substitution may be selected from the group consisting of A1C, A1G, A1H, N21C, N21D, N21Q, N35C, N35D, N35H, N35Q, N64C, N64D, N64Q, N64W, Q113C, Q113R, K114C and K114R. N35Q and N64Q are particularly advantageous. Surprisingly, substitutions as employed in the invention do not adversely affect IL-22 activity.

Particular combinations of additional substitutions include (i) A1G, N21D, N35D and N64D; (ii) A1G, N35Q and N64Q; (iii) A1G and N64C; (iv) A1G and Q113C; (v) A1G and K114C; (vi) A1G and M25L; (vii) A1G and M52L; (viii) A1G and M139L; (ix) A1G and N36Q; (x) A1G and D117E; (xi) A1G and N21Q; (xii) A1G and N35Q; (xiii) A1G and N64Q; (xiv) A1G, N21Q and N35Q; (xv) A1G, N21Q and N64Q; (xvi) A1G, N21Q, N35Q and N64Q; (xvii) A1G and K11C; (xviii) A1G and N13C; (xix) N35Q and N64Q; (xx) A1C, N35Q and N64Q; (xxi) H6C, N35Q and N64Q; (xxii) I3C, N35Q and N64Q; (xxiii) P2C, N35Q and N64Q; (xxiv) L32C, N35Q and N64Q; (xxv) N35Q, M52C and N64Q; (xxvi) N13C, N35Q and N64Q; (xxvii) N21C, N35Q and N64Q; (xxviii) N35Q, N64Q and N94C; (xxix) N35Q, N64Q and P73C; (xxx) N35Q, N64Q and Q113C; (xxxi) N35Q, N64Q and R91C; (xxxii) N35Q, N64Q and R110C; (xxxiii) S12C, N35Q and N64Q; (xxxiv) N35Q, S51C and N64Q; (xxxv) N35Q, S53C and N64Q; (xxxvi) N35Q, T37C and N64Q; (xxxvii) N35Q, N64Q and T98C; (xxxviii) Q15C, N35Q and N64Q; (xxxix) N35C and N64Q; (xxxx) H6C, N35Q and N64Q; and (xxxxi) A33C, N35Q and N64Q. Any and all combinations of substitutions are envisaged and form part of the invention.

A derivative of the first aspect comprises a substitution at position 95 and/or 106. Advantageously the substitution is R95C or L106C. The derivative may typically comprise an additional amino acid substitution whereby Cys is substituted for a native residue, optionally in any of the positions identified above, such as position 1, 2, 3, 6, 11, 12, 13, 15, 21, 32, 33, 35, 37, 51, 52, 53, 63, 64, 71, 73, 91, 94, 98, 110, 113, 114 and/or 127. An R95C or L106C substitution combined with substitutions in two glycosylation sites at positions 35 and 64 is particularly advantageous, as it leads to faster uptake without adversely affecting potency or half-life (see Derivatives 1 and 2 in Example 1). In one advantageous embodiment, a derivative of the first aspect comprises the substitutions, N35Q, N64Q and R95C. In another advantageous embodiment, a derivative of the first aspect comprises the substitutions, N35Q, N64Q and L106C.

The variations within the native sequence may also include amino acid insertions. Up to five, 10, 15, 20, 25, 30, 35, 40, 45 or even up to 50 amino acids may be inserted within the native sequence. Trimers, pentamers, septamers, octamers, nonamers and 44-mers are particularly advantageous in this regard. Exemplary sequences are shown in Table 1. Insertions can be made at any location in the native sequence, but those in helix A (for example, at residue 30), loop CD (for example, at residue 75), helix D (for example, at residue 85) and/or helix F (for example, at residue 124) are preferred.

**Table 1: Sequence of exemplary amino acid insertions**

| **n-mer** | **Exemplary amino acid sequence** | **SEQ ID NO.** |
|---|---|---|
| Trimer | E-T-S | n/a |
| Pentamer | R-V-Q-F-Q or C-V-E-I-P | 2,3 |
| Septamer | G-S-G-S-G-S-C | 4 |
| Octamer | I-E-A-L-T-P-H-S or Y-G-Q-R-Q-W-K-N | 5,6 |
| Nonamer | V-F-I-I-N-N-S-L-E | 7 |
| 44-mer | R-A-A-S-A-G-S-Y-S-E-W-S-M-T-P-R-F-T-P-W-W-E-T-K- I-D-P-P-V-M-N-I-T-Q-V-N-G-S-L-L-V-I-L-H | 8 |

Sequence variations relative to the amino acid sequence of hIL-22, if present, typically include an extension, such as the addition of a peptide at the N-terminal end. The peptide may consist of up to five, 10, 15, 20, 25, 30, 35, 40, 45 or even up to 50 amino acids. Monomers, trimers, octamers, 13-mers, 15-mers, 16-mers, 21-mers, 28-mers are particularly advantageous in this regard. Exemplary sequences are shown in Table 2. In an embodiment, the IL-22 protein included in a derivative of the first aspect comprises an N-terminal G-P-G.

**Table 2: Sequence of exemplary N-terminal peptides**

| **n-mer** | **Exemplary amino acid sequence** | **SEQ ID NO.** |
|---|---|---|
| Monomer | C, G or M | n/a |
| Trimer | G-P-G | n/a |
| Pentamer | A-E-P-E-E | 9 |
| Heptamer | G-P-A-E-P-E-E | 10 |
| 12-mer | G-S-G-S-G-S-G-S-G-S-G-S | 11 |
| 13-mer | G-G-S-S-G-S-G-S-E- V-L-F-Q | 12 |
| 14-mer | G-P-G-S-G-S-G-S-G-S-G-S-G-S | 13 |
| 14-mer | G-G-S-S-G-S-G-S-E-V-L-F-Q-G | 14 |
| 16-mer | G-G-S-S-G-S-G-S-E-V-L-F-Q-G-P-G | 15 |
| 20-mer | G-G-S-S-G-S-G-S-E-V-L-F-Q-G-P-A-E-P-E-E | 16 |
| 27-mer | G-G-S-S-G-S-G-S-E-V-L-F-Q-G-P-G-S-G-S-G-S-G-S-G-S- G-S | 17 |

Sequence variations relative to the amino acid sequence of hIL-22, if present, may include the addition of a peptide at the C-terminal end. The peptide may consist of up to five, 10, 15, 20, 25, 30, 35, 40, 45 or even up to 50 amino acids. Exemplary C-terminal peptide sequences include those shown in Table 2 (for N-terminal peptides). A septamer is particularly advantageous in this regard, optionally having the amino acid sequence, G-S-G-S-G-S-C (SEQ ID NO. 18).

The derivatives of the invention may include both an N-terminal and a C-terminal peptide in addition to the variant hIL-22 amino acid sequence as herein described. Any combination of the N- and C-terminal peptides described herein is envisaged and expressly included in the invention.

It will be appreciated that the invention extends to any derivative of IL-22, which comprises a fatty acid covalently attached to a variant of hIL-22, said variant comprising a substitution at position 95 or 106 of hIL-22, and the fatty acid is covalently attached at said substituted position. The "variant" can be a protein having at least 10% sequence identity with hIL-22. In an embodiment, the variant has at least 20%, or even at least 30%, sequence identity with hIL-22. The variant may have "substantially the amino acid sequence" of hIL-22, which can mean a sequence that has at least 40% sequence identity with the amino acid sequence of hIL-22. Accordingly, in an embodiment, a derivative of the first aspect has at least 50%, at least 60%, at least 70%, at least 80%, at least 90%, at least 95% or at least 99% amino acid sequence identity with hIL-22. Exemplary IL-22 protein variants, which are incorporated in the particular derivatives of the invention disclosed in the experimental section, are set forth in SEQ ID NOs. 19 and 20.

The skilled technician will appreciate how to calculate the percentage identity between two amino acid sequences. An alignment of the two sequences must first be prepared, followed by calculation of the sequence identity value. The percentage identity for two sequences may take different values depending on: (i) the method used to align the sequences, for example, ClustalW, BLAST, FASTA, Smith-Waterman (implemented in different programs), or structural alignment from 3D comparison; and (ii) the parameters used by the alignment method, for example, local *versus* global alignment, the pair-score matrix used (for example, BLOSUM62, PAM250, Gonnet etc.) and gap-penalty, for example, functional form and constants.

Having made the alignment, there are many different ways of calculating percentage identity between the two sequences. For example, one may divide the number of identities by: (i) the length of shortest sequence; (ii) the length of alignment; (iii) the mean length of sequence; (iv) the number of non-gap positions; or (iv) the number of equivalenced positions excluding overhangs. Furthermore, it will be appreciated that percentage identity is also strongly length-dependent. Therefore, the shorter a pair of sequences is, the higher the sequence identity one may expect to occur by chance.

Hence, it will be appreciated that the accurate alignment of amino acid sequences is a complex process. The popular multiple alignment program ClustalW [48,49] is a preferred way for generating multiple alignments of proteins in accordance with the invention. Suitable parameters for ClustalW may be as follows: For protein alignments: Gap Open Penalty = 10.0, Gap Extension Penalty = 0.2, and Matrix = Gonnet. For DNA and Protein alignments: ENDGAP = -1, and GAPDIST = 4. Those skilled in the art will be aware that it may be necessary to vary these and other parameters for optimal sequence alignment.

Preferably, calculation of percentage identities between two amino acid sequences may then be calculated from such an alignment as (N/T)^{∗}100, where N is the number of positions at which the sequences share an identical residue, and T is the total number of positions compared including gaps but excluding overhangs. Hence, a most preferred method for calculating percentage identity between two sequences comprises (i) preparing a sequence alignment using the ClustalW program using a suitable set of parameters, for example, as set out above; and (ii) inserting the values of N and T into the following formula: Sequence Identity = (N/T)^{∗}100.

Alternative methods for identifying similar sequences will be known to those skilled in the art.

Suitably, a derivative of the first aspect comprises 200 amino acids or less. For example, the derivative comprises less than 190, less than 180, less than 170, less than 160 or even less than 150 amino acids. Suitably, the derivative will comprise at least 146 amino acids, however, this being the number of amino acids in hIL-22. It may comprise at least 150 amino acids, at least 160 amino acids, at least 170 amino acids or even at least 180 amino acids. The derivatives of the invention can comprise proteins of any length within the above ranges, but they will typically be 146-180 amino acids in length.

The derivatives of the invention, having the herein described variant amino acid sequence, include a fatty acid covalently attached to the IL-22 protein at position 95 or 106. The fatty acid is typically covalently attached to the IL-22 protein by a linker. The fatty acid and linker are suitably connected to each other via an amide bond, and the linker is covalently attached to the IL-22 protein. The fatty acid and linker may thus be present as a side chain on the IL-22 protein. It was surprising to the inventors that a covalently attached fatty acid does not adversely affect IL-22 activity. It was particularly surprising that fatty acid attachment is associated with additional advantages, such as prolongation of half-life.

The fatty acid may be any suitable fatty acid. In particular, the fatty acid may be of Formula I:

HOOC-(CH₂)ₓ-CO-*,

wherein x is an integer in the range of 10-18, optionally 12-18, 14-16 or 16-18, and ^{∗} designates a point of attachment to the IL-22 protein or linker. It may be a fatty diacid, such as a C12, C14, C16, C18 or C20 diacid. Advantageously, the fatty acid is a C16 or C18 diacid, and most advantageously it is a C18 diacid.

For example, -(CH₂)ₓ- in Formula I may be a straight alkylene in which x is 10. This fatty acid may be conveniently referred to as C12 diacid, i.e. a fatty di-carboxylic acid with 12 carbon atoms. Alternatively, -(CH₂)ₓ- in Formula I may be a straight alkylene in which x is 12. This fatty acid may be conveniently referred to as C14 diacid, i.e. a fatty di-carboxylic acid with 14 carbon atoms. In a similar fashion, -(CH₂)ₓ- in Formula I may be a straight alkylene in which x is 14 (C16 diacid), 16 (C18 diacid) or 18 (C20 diacid). Suitably, a derivative of the first aspect includes a C14, C16, C18 or C20 diacid; more suitably, a C16 or C18 diacid, and even more suitably a C18 diacid.

Alternatively, the fatty acid may be of Formula II:

H₃C-(CH₂)ₓ-CO-*,

wherein x is an integer in the range of 10-18, optionally 12-18, 14-16 or 16-18, and ^{∗} designates a point of attachment to the IL-22 protein or linker. It may be a fatty monoacid, such as a C12, C14, C16, C18 or C20 monoacid. Advantageously, the fatty monoacid is a C16 or C18 monoacid, and most advantageously it is a C16 monoacid.

For example, -(CH₂)ₓ- in Formula II may be a straight alkylene in which x is 10. This fatty acid may be conveniently referred to as C12 monoacid, i.e. a fatty mono-carboxylic acid with 12 carbon atoms. Alternatively, -(CH₂)ₓ- in Formula II may be a straight alkylene in which x is 12. This fatty acid may be conveniently referred to as C14 monoacid, i.e. a fatty mono-carboxylic acid with 14 carbon atoms. In a similar fashion, -(CH₂)ₓ- in Formula II may be a straight alkylene in which x is 14 (C16 monoacid), 16 (C18 monoacid) or 18 (C20 monoacid). Suitably, a derivative of the first aspect includes a C14, C16, C18 or C20 monoacid; more suitably, a C16 or C18 monoacid, and even more suitably a C16 monoacid.

The diacid or monoacid may be capable of forming non-covalent associations with albumin, thereby promoting circulation of the derivative in the blood stream. The shorter diacids and monoacids (e.g. C16 diacid or monoacid) have lower albumin affinity and thus a shorter half-life than the longer diacids and monoacids (e.g. C18 diacid and monoacid). However, they are still long acting derivatives with an expected half-life in man of over one day.

The monoacids are also lipophilic, which means they tend to bind to bio-membranes. This non-albumin dependent protraction through integration in bio-membranes may form a local reservoir, thus ensuring a prolonged local action. This could be hypothesised to provide an advantage in topical administration, oral administration, administration as rectal suppositories or rectal foams or pulmonary inhalation.

Fatty acid attachment will, in itself, also stabilise the IL-22 protein against proteolytic degradation. The resulting half-life is typically similar to that of IL-22-Fc fusions (i.e. greatly improved compared to hIL-22).

The derivatives of the first aspect may comprise particular combinations of a fatty acid and IL-22 protein. For example, a C14, C16, C18 or C20 diacid or monoacid may be attached to an IL-22 protein comprising a Cys residue at position 95 or 106 of hIL-22. In one example, a derivative of the first aspect comprises a C18 diacid and the IL-22 protein comprises a Cys residue substituted at position 95 or 106 of hIL-22. In another example, a derivative of the first aspect comprises a C18 diacid and the IL-22 protein comprises a Cys residue substituted at position 95 or 106 of hIL-22 and a Gln residue substituted at positions 35 and 64 of hIL-22 (such as in Derivatives 1 and 2). In these examples, the IL-22 protein may additionally comprise an N- or C- terminal pentamer having the sequence, A-E-P-E-E (SEQ ID NO. 9).

As above, the fatty acid is suitably connected to a linker, which is attached to the IL-22 protein at position 95 or 106. The linker may comprise several linker elements, including one or more amino acids such as one or more Glu and/or Lys residues. The linker may include an oxyethylene glycine unit or multiple linked oxyethylene glycine units, optionally 2-5 such units, advantageously 2 units. One or more OEG residues, C₂DA and/or Ac groups may alternatively or additionally be included. The linker may comprise a Cys-reactive unit. A "Cys-reactive unit", as used herein, can mean a functional unit that is able to react with the sulphur atom of a Cys to create a carbon-sulphur covalent bond. The Cys-reactive unit can have any of several forms, but suitably includes a carbon atom attached to a leaving group, which leaving group becomes displaced by the sulphur atom of the Cys during formation of the carbon-sulphur bond. The leaving group may be a halogen, optionally a bromine atom. This bromide leaving group can be alpha to an Ac functional group; advantageously it is a bromo-Ac functional group. The leaving group may alternatively be a functionalised hydroxyl group of the form mesylate or tosylate, or an unfunctionalised hydroxyl group. Further, the leaving group can be a maleimide or other functional group. Exemplary linkers include γGlu-OEG-OEG-C₂DA-Ac, γGlu-γGlu-γGlu-γGlu-OEG-OEG-εLys-αAc and γGlu-OEG-OEG-εLys-αAc, but any suitable linker may be employed. In a particular embodiment, the linker is γGlu-OEG-OEG-C₂DA-Ac.

The fatty acid, or linker, is attached to a substituted amino acid residue at position 95 or 106 in the IL-22 protein. The native residue is typically substituted with Cys or Lys to enable attachment of the fatty acid or linker. In particular, the fatty acid or linker may be attached to a Cys residue substituted at position 95 or 106 of hIL-22.

The attachment of the fatty acid or linker to the IL-22 protein is a covalent attachment. For example, a Cys-reactive fatty acid or linker may be used to attach the fatty acid or linker to a Cys residue in the IL-22 protein. The fatty acid or linker may be covalently attached to the sulphur atom of the Cys residue via a thioether bond. Alternatively, a Lys-reactive fatty acid or linker may be used to attach the fatty acid or linker to a Lys residue in the IL-22 protein. The fatty acid or linker may alternatively be covalently attached to the free amine (-NH2) group in the N-terminus of the IL-22 protein (irrespective of the amino acid in position 1). Attachment can proceed as with Cys attachment, albeit with sub-stoichiometric amounts of fatty acid or linker containing a suitable N-reactive species. The fatty acid or linker may be presented in the form of an aldehyde (the N-reactive species) and be covalently attached to the free amine employing a classically known reductive amination.

A derivative of the first aspect thus suitably comprises a C14, C16, C18 or C20 diacid or monoacid attached by a linker to a variant of hIL-22, wherein the variant comprises a substitution at position 95 or 106 of hIL-22, and the linker is covalently attached at said substituted position. Advantageously, a Cys residue is substituted at position 95 or 106 of hIL-22 and the linker is attached to the Cys residue. Optionally a Gln residue is substituted at positions 35 and 64.

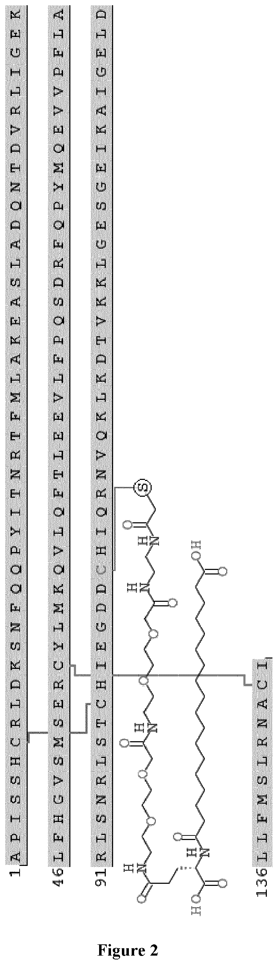

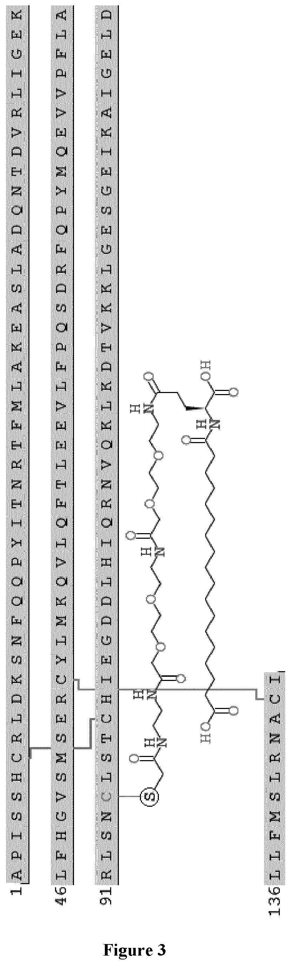

Exemplary derivatives of the first aspect comprise an IL-22 protein as set forth in SEQ ID NO. 19 or 20. Particularly advantageous derivatives are shown in Table 3, illustrated in Figures 2 and 3 and exemplified herein.

**Table 3: Exemplary derivatives of IL-22**

| **ID** | **Sequence variations** | **SEQ ID NO.** | **Covalent attachment site** | **Linker** | **Fatty acid** |
|---|---|---|---|---|---|
| Derivative 1 | N35Q, N64Q, L106C substitutions | 19 | 106C | γGlu-OEG-OEG-C₂DA-Ac | C18 diacid |
| Derivative 2 | N35Q, N64Q, R95C substitutions | 20 | 95C | γGlu-OEG-OEG-C₂DA-Ac | C18 diacid |

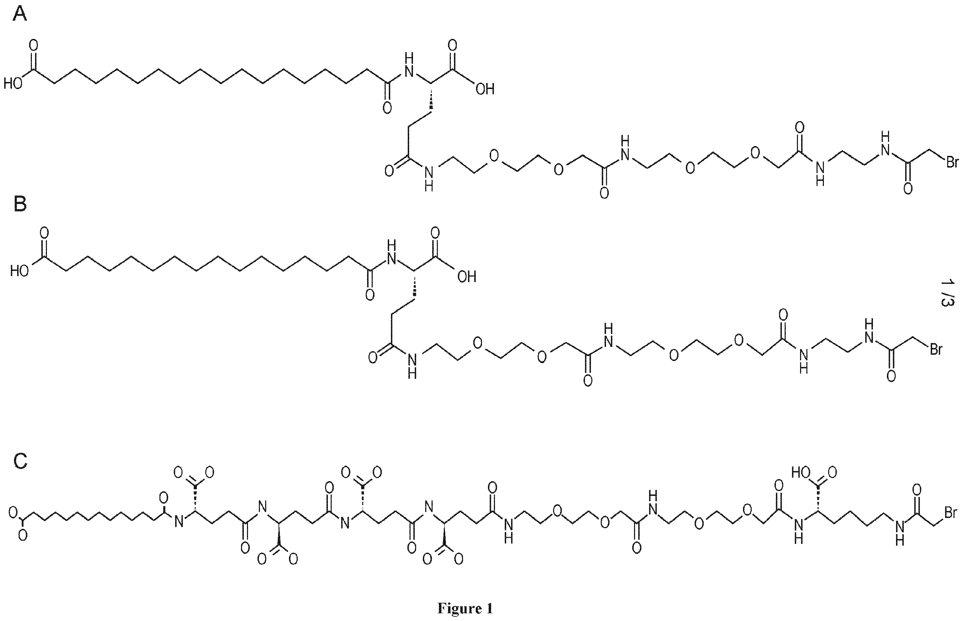

Figure 1A illustrates a C18 diacid connected to a linker comprising a Cys-reactive unit. This is the fatty acid and linker (side chain) used in Derivatives 1 and 2. Figure 1B illustrates a C16 diacid connected to a linker comprising a Cys-reactive unit. Figure 1C illustrates a C14 diacid connected to a linker comprising a Cys-reactive unit.

Derivatives 1 and 2 are illustrated in Figures 2 and 3, respectively.

The derivatives of the invention may exist in different stereoisomeric forms and the invention relates to all of these.

According to a second aspect of the invention, there is provided a process for preparing a derivative of the first aspect comprising covalently attaching a fatty acid to an IL-22 protein, wherein the IL-22 protein is a variant of hIL-22, said variant comprising a substitution at position 95 or 106 of hIL-22, and the process comprises covalently attaching the fatty acid at said substituted position.

The process may be used to produce any of the different derivatives of IL-22 described or envisaged herein, but it is particularly advantageous when a fatty acid is covalently attached to a variant IL-22 protein. The IL-22 protein employed in the second aspect is a substituted form of hIL-22, wherein the native residue at position 95 and/or 106 is replaced by an alternative amino acid. The variant hIL-22 protein is optionally further substituted at position 1, 21, 35, 64, 113 and/or 114. Exemplary substitutions include A1C, A1G, A1H, N21C, N21D, N21Q, N35C, N35D, N35H, N35Q, N64C, N64D, N64Q, N64W, R95C, L106C, Q113C, Q113R, K114C and/or K114R. Preferably, the IL-22 protein is substituted with a Cys residue at position 95 and/or 106, and a Gln residue at position 35 and/or 64.

The fatty acid can be obtained by any means known in the art, including recombinant means. Suitable fatty acids are commercially available or readily derived from available starting materials using standard chemical synthesis.

The IL-22 protein can be obtained by any means known in the art, including recombinant means. The production of recombinant hIL-22 has been previously described and is well-known in the art. Desired variant IL-22 proteins can be produced in a similar manner. An experienced investigator in the field would be readily able to identify suitable nucleic acid sequences that encode the desired variant IL-22 proteins. The skilled person would hence be readily able to execute this part of the invention, based upon the existing knowledge in the art. Suitably, the IL-22 proteins are produced in mammalian systems, such as in Chinese hamster ovary (CHO) cells, using standard techniques. A polyhistidine tag (His-tag) may be employed to aid affinity purification of the recombinant proteins.

In this regard, IL-22 proteins as used in the invention can be prepared using a post expression cleavable His-tag - an N- or C-terminal addition of less than 10, preferably six, histidine residues that can be purified by affinity to a nickel column. The His-tag is linked to the Nor C-terminal of the protein via a linker that can be digested by a known protease to leave the free IL-22 protein. The cleavable His-tag can have the amino acid sequence, HHHHHHGGSSGSGSEVLFQ (SEQ ID NO. 21), and the protease-cleavable linker can be a tobacco etch virus (TEV) linker, whose consensus sequence for the native cut sites is ENLYFQ\S (SEQ ID NO. 22), where '\' denotes the cleaved peptide bond or a human rhinovirus-14 3C (HRV14-3C) protease cleavable linker with EVLFQ consensus cleavage site. Cleavage may be achieved by incubating approximately 10 µg protease with 2.5 µg protein and 10 mM 2-mercaptoethanol at room temperature for 4h.

To further illustrate the invention, a representative process for protein preparation is provided as follows. The process involves preparing a plasmid DNA that encodes the desired amino acid sequence of the IL-22 protein. This plasmid can be transiently transfected into a cell line, for example CHO-K1, which is allowed to grow in a relevant medium before growth is increased by the addition of a known enhancer. The secreted IL-22 protein can then be harvested through known methods of centrifugation and sterile filtration before the protein is purified on a nickel column. Following concentration and buffer exchange the His-tag is removed using a HRV14-3C protease before alkylation with a fatty acid (described further below) and final purification and buffer exchange. Analysis of the final product using SDS-PAGE, size exclusion chromatography or liquid chromatography with tandem mass spectrometry (LC-MS-MS) with, or without, deglycosylation can be used to ensure the quality of the final product.

The fatty acid can be covalently attached to the IL-22 protein either directly or using a linker as described for the first aspect. The linker can be obtained by any means known in the art. A representative method for preparing the fatty acid and linker, if employed, is as follows (exemplified by a C16 diacid, but any derivative could be made using a similar method).

A solution of N-(benzyloxycarbonyloxy) succinimide (100 g, 401 mmol) in dichloromethane (500 ml) is added to a solution of ethylene diamine (189 ml, 2.81 mol) in dichloromethane (750 ml). After 30 minutes the suspension is filtered, washed and concentrated *in vacuo.* The residue is diluted with toluene (750 ml), washed and extracted with dichloromethane (4 x 200 ml), dried over anhydrous sodium sulphate, filtered, concentrated *in vacuo* and diluted with hexanes (200 ml). A 4 M solution of hydrogen chloride in ether (100 ml, 400 mmol) is added to the solution, the resulting suspension concentrated *in vacuo* and diluted with hexanes (1 1). The precipitated solid is filtered, washed with hexanes and dried *in vacuo* to give (2-aminoethyl) carbamic acid benzyl ester hydrochloride as a white powder.

2-Chlorotrityl resin 100-200 is loaded with {2-[2-(9H-fluoren-9-ylmethoxycarbonylamino)-ethoxy]-ethoxy}-acetic acid (Fmoc-Ado-OH, 17.5 g, 45.4 mmol) The Fmoc group is removed and a solution of 0-6-chloro-benzotriazol-1-yl)-N,N,N',N'-tetramethyluronium tetrafluoroborate (TCTU, 24.2 g, 68.1 mmol) and N,N-diisopropylethylamine (21.4 ml, 123 mmol) in N,N dimethylformamide (140 ml) is added to the resin and the mixture shaken for one hour. The resin is filtered and washed. The Fmoc group is removed by treatment with 20% piperidine as before. The resin is washed as before.

A solution of (S)-2-(fluoren-9-ylmethoxycarbonylamino)-pentanedioic acid 1-tert-butyl ester (Fmoc-Glu-OtBu, 29.0 g, 68.1 mmol), TCTU (24.2 g, 68.1 mmol) and N,N-diisopropylethylamine (21.4 ml, 123 mmol) in N,N dimethylformamide (140 ml) is added to the resin and the mixture shaken for one hour. The resin is filtered and washed as before. The Fmoc group is removed by treatment with 20% piperidine as before. The resin is washed as before.

A solution of 16-tert-butoxy)-16-oxohexadecanoic acid (23.3 g, 68.1 mmol), TCTU (24.2 g, 68.1 mmol) and N,N diisopropylethylamine (21.4 ml, 123 mmol) in N,N-dimethylformamide/dichloromethane mixture (4:1, 200 ml) is added to the resin. The resin is shaken for one hour, filtered and washed with N,N-dimethylformamide (3 x 250 ml), dichloromethane (2 x 250 ml), methanol (2 x 250 ml) and dichloromethane (6 x 250 ml). The product is cleaved from the resin by treatment with 2,2,2-trifluoroethanol (250 ml) for 18 hours. The resin is filtered off and washed with dichloromethane (2 x 250 ml), 2-propanol/dichloromethane mixture (1:1, 2 x 250 ml), 2-propanol (250 ml) and dichloromethane (3 x 250 ml).

The solutions are combined, the solvent is evaporated and crude product purified by flash column chromatography. Pure (S)-22-(tert-butoxycarbonyl)-41,41-dimethyl-10,19,24,39-tetraoxo-3,6,12,15,40-pentaoxa-9,18,23-triazadotetracontanoic acid is dried *in vacuo* and obtained as a pale yellow thick yellow oil.

2-(7-Aza-1H-benzotriazole-1-yl)-1,1,3,3 -tetramethyluronium hexafluorophosphate (HATU, 11.4 g, 30.1 mmol) and triethylamine (8.77 ml, 62.9 mmol) are subsequently added to a solution of (S)-22-(tert-butoxycarbonyl)-41,41-dimethyl-10,19,24,39-tetraoxo-3,6,12,15,40-pentaoxa-9,18,23-triazadotetracontanoic acid (22.4 g, 27.4 mmol) in dry dichloromethane (110 ml). Triethylamine (72 ml, 41.0 mmol) is added to a suspension of (2-amino-ethyl)-carbamic acid benzyl ester hydrochloride (6.94 g, 30.1 mmol) in dry dichloromethane (165 ml) and the resulting mixture is added to the above solution. The mixture is stirred at room temperature overnight and then evaporated to dryness. The residue is re-dissolved and washed; dried over anhydrous sodium sulphate and evaporated column chromatography (Silicagel 60, 0.040-0.060 mm; eluent: dichloromethane/methanol 95:5) to afford 15-[(S)3-(2-{2-[(2-{2-[(2-benzyloxycarbonylamino-ethylcarbamoyl)-methoxy]-ethoxy}ethylcarbamoyl)methoxy]ethoxy)-ethylcarbamoyl)-1-tert-butoxycarbonylpropylcarbamoyl]-pentadecanoic acid tert-butyl ester as a pale yellow thick oil.

Palladium on carbon (10%, 1.27 g, 1.20 mmol) is added to a solution of the above compound (23.8 g, 24.0 mmol) in methanol (350 ml) and the resulting mixture hydrogenated at normal pressure for four hours. The catalyst is filtered off and the filtrate evaporated to dryness. The residue is evaporated several times from dichloromethane in order to remove residues of methanol and dried *in vacuo* to yield tert-butyl(S)-1-amino-25-tert-butoxycarbonyl)-4,13,22,27-tetraoxo-6,9,15,18-tetraoxa-3,12,21,26-tetraazadotetracontan-42-oate as a thick colourless oil.

N,N-Diisopropylethylamine (4.98 ml, 28.6 mmol) is added to a solution of the above amine (20.5 g, 23.8 mmol) in dry dichloromethane (290 ml) at - 30° C. under argon. Bromoacetyl bromide (2.48 ml, 28.6 mmol) is added dropwise and the resulting solution is stirred at -30° C for an additional three hours. The cooling bath is removed, the mixture is stirred at room temperature for one hour, and the solvent is removed *in vacuo.* The residue is re-dissolved in ethyl acetate (450 ml) and washed with 5% aqueous solution of citric acid (300 ml). The phases are separated within one hour. The organic layer is left to separate overnight to give three phases. The clear aqueous layer is removed and the residual two phases shaken with a saturated aqueous solution of potassium bromide (100 ml). The phases are left to separate overnight, the aqueous phase removed and the organic phase dried over anhydrous sodium sulphate. The solvent is removed *in vacuo* and the residue purified by flash column chromatography: dichloromethane/methanol 95:5) to afford tertbutyl(S)-1-bromo-28-tert-butoxycarbonyl)-2,7,16,25,30-pentaoxo-9,12,18,21-tetraoxa-3,6,15,24,29-pentaazapenta-tetracontan-45-oate as a colourless solid.

The above compound (19.5 g, 19.8 mmol) is dissolved in trifluoroacetic acid (120 ml) and the resulting solution is stirred at room temperature for 1.5 hours. Trifluoroacetic acid is removed *in vacuo* and the residue is evaporated from dichloromethane (6 x 200 ml). Diethyl ether (200 ml) is added to the oily residue and the mixture stirred overnight to give a suspension. The solid product is filtered, washed with diethyl ether and hexanes and dried *in vacuo* to afford the desired product 15-{(S)-1-carboxy3-[2-(2-{[2-(2-{[2-(2-Bromoacetylamino)ethylcarbamoyl]methoxy}-ethoxyethyl-carbamoyl]methoxy}ethoxylethylcarbamoyl]propylcarbamoyl}pentadecanoic acid as a white powder.

Covalent attachment of the fatty acid or linker to the IL-22 protein may be carried out using standard procedures in the art. The linker, if employed, thus enables covalent attachment of the IL-22 protein to the fatty acid. By way of a non-limiting example, a Cys-reactive fatty acid or linker may be reacted with the sulphur atom of a Cys residue in the IL-22 protein, so forming a thioether bond. Suitable conditions for the covalent attachment step may be exemplified as follows: Tris in water is added to IL-22 protein (70 mg) in Tris and NaCl-buffer (1.35 mg/ml), to adjust to pH 8. Bis(p-sulfonatophenyl)- phenylphosphine dihydrate dipotassium (BSPP) salt (12 mg), dissolved in water, is added and stirred gently for four hours at room temperature. 15-{(S)-1-Carboxy-3-[2-(2-{[2-(2-{[2-(2-bromoacetylamino)-ethylcarbamoyl]ethoxy}ethoxy)ethylcarbamoyl]methoxy}ethoxy)ethylcarbamoyl]propylcarbamoyl}pentadecanoic acid (19 mg, 0.022 mmol) in ethanol (0.5 ml) is added and the mixture stirred gently overnight. MiliQ water (150 ml) is added to lower the conductivity to 2.5 mS/cm. The mixture is then purified using anion exchange on a MonoQ 10/100 GL column using binding buffer (20 mM Tris, pH 8.0), elution buffer (20 mM Tris, 500 mM NaCl, pH 8.0), flow 6 ml and a gradient of 0-80% elution buffer over 60 column volumes. The derivatives of the invention may be purified using any suitable procedure known in the art, such as chromatography, electrophoresis, differential solubility or extraction.

As described herein, the inventors were surprised to find that fatty acids could be covalently attached to an IL-22 protein whilst maintaining biological activity. It was particularly surprising that such a minimal modification to IL-22 could result in high potency (close to hIL-22) combined with a very long circulatory half-life. This particular combination of properties may be highly desirable.

The potency of the derivatives may be determined in an *in vitro* assay with whole cells expressing human IL-22 receptors. For example, the response of the human IL-22 receptors may be measured using baby hamster kidney (BHK) cells overexpressing IL-22R1, IL-10R2 and a phospho-STAT3 (pSTAT3) responsive reporter gene. Alternatively, HepG2 cells endogenously expressing the IL-22 receptor may be used. Activation of the receptors leads to activation of the STAT3 signaling pathway, which can be measured using a luciferase reporter gene with a STAT3-induced promoter or by assaying pSTAT3, for example. *In vivo* potency may be determined in animal models or in clinical trials, as is known in the art.

The half maximal effective concentration (EC₅₀) value is often used as a measure of the potency of a drug. As this represents the concentration of drug required to produce 50% of the maximal effect, the lower the EC₅₀ value, the better the potency. The derivatives of the invention suitably have a potency (EC₅₀ value) measured using IL-22 receptor-mediated STAT3 activation in cells of below 1.5 nM, below 1.25 nM, below 1 nM, below 0.75 nM, below 0.5 nM, below 0.25 nM or even below 0.1 nM (e.g. determined as described in Example 1). The derivatives of the invention suitably have a potency (EC₅₀ value) measured by assaying pSTAT3 in cells of below 15 nM, below 12 nM, below 10 nM, below 7 nM or even below 5 nM.

Advantageously, the potency of the derivatives of IL-22 may be higher than that of IL-22-Fc fusions. For example, Genentech has reported a 34-fold reduction in *in vitro* potency for its IL-22-Fc fusion, UTTR1147A, compared to hIL-22 (Stefanich et al., Biochem Pharmacol, 2018, 152:224-235). By contrast, covalent attachment of a fatty acid to a variant hIL-22 has been shown to cause only a three- or five-fold reduction in potency (see Derivatives 1 and 2 in Example 1). Whilst both IL-22-Fc fusions and the derivatives of the present invention may be comparable in terms of their improved half-life over hIL-22 and biological function in at least some settings, the derivatives of the invention may have the additional advantage of minimal loss of potency.

The circulatory elimination half-life (T_{1/2}) of the derivatives may be determined *in vivo* by administering the derivatives subcutaneously or intravenously in a suitable animal model, such as a mouse, rat or minipig. Suitable methods will be known to the skilled person. By way of a non-limiting example, the derivatives of the first aspect have a circulatory half-life after subcutaneous or intravenous administration to mice of at least one hour, at least three hours, at least five hours or even at least eight hours. The derivatives may have a circulatory half-life after subcutaneous or intravenous administration to rats of at least three hours, at least five hours, at least eight hours, at least 10 hours or even at least 13 hours. The derivatives may have a circulatory half-life after subcutaneous or intravenous administration to minipigs of at least 25 hours, at least 40 hours, at least 70 hours or even at least 100 hours.

The inventors have also found that the derivatives of the invention are absorbed rapidly *in vivo.* Advantageously, absorption of the derivatives following subcutaneous dosing may occur faster than that of IL-22-Fc fusions. Mean absorption time is an accurate parameter for measuring uptake because it is independent of dose and maximum plasma concentration following drug administration. It can be calculated based upon mean residence time, i.e. the time that a drug spends in the body prior to elimination once absorption has been completed. The derivatives of the invention suitably have a mean absorption time in pigs of below 100 h, below 90 h, below 80 h, below 70 h or even below 60 h.

The derivatives of the invention also have good biophysical properties, such as high physical stability and/or solubility, which may be measured using standard methods in the art. In fact, monoacid derivatised variants have significantly altered biophysical properties compared to hIL-22, which improves their therapeutic potential. Of note, they are stabilised against proteolytical breakdown and renal clearance through binding to albumin and interaction with lipid membranes, for example. Importantly, the inventors have found that conjugation of monoacid-containing fatty acid moieties can be carried out at select sites of the IL-22 protein backbone, without compromising the activity of the conjugated derivative, as measured *in vitro.*

Therefore, according to a third aspect of the invention, there is provided a pharmaceutical composition comprising a derivative of the first aspect and a pharmaceutically acceptable vehicle.

A pharmaceutical composition of the third aspect may comprise any of the different derivatives of IL-22 described or envisaged herein. Suitably, it comprises one of the derivatives of IL-22 identified herein as Derivative 1 or 2.

A derivative of the first aspect, or a pharmaceutical composition of the third aspect, will suitably demonstrate increased circulatory elimination half-life compared to hIL-22. Advantageously it will demonstrate increased circulatory elimination half-life compared to hIL-22 by at least 50%, at least 75%, at least 100% or more.

The pharmaceutical compositions of the third aspect may be prepared by combining a therapeutically effective amount of a derivative of the first aspect with a pharmaceutically acceptable vehicle. The formulation of pharmaceutically active ingredients with various excipients is known in the art.

A "therapeutically effective amount" of a derivative of the first aspect is any amount which, when administered to a subject, is the amount of derivative that is needed to treat the disease, disorder or condition or produce the desired effect.

For example, the therapeutically effective amount of derivative used may be from about 0.001 mg to about 1000 mg, and preferably from about 0.01 mg to about 500 mg. It is preferred that the amount of derivative is an amount from about 0.1 mg to about 100 mg, and most preferably from about 0.5 mg to about 50 mg.

A "pharmaceutically acceptable vehicle" as referred to herein, is any known compound or combination of known compounds that are known to those skilled in the art to be useful in formulating pharmaceutical compositions.

In one embodiment, the pharmaceutically acceptable vehicle may be a solid; optionally the composition may be in the form of a powder for resuspension. A solid pharmaceutically acceptable vehicle may include one or more substances which may also act as flavouring agents, lubricants, solubilisers, suspending agents, dyes, fillers, glidants, inert binders, preservatives or dyes. The vehicle may also be an encapsulating material. In powders, the vehicle is a finely divided solid that is in admixture with the finely divided derivatives according to the invention. The powders preferably contain up to 99% derivative. Suitable solid vehicles include, for example calcium phosphate, magnesium stearate, talc, sugars, lactose, dextrin, starch, gelatin, cellulose and ion exchange resins.

In another embodiment, the pharmaceutical vehicle may be a gel and the composition may be in the form of a cream or the like.

However, the pharmaceutical vehicle may be a liquid; optionally the pharmaceutical composition is in the form of a solution. Liquid vehicles are used in preparing solutions, suspensions, emulsions, syrups, elixirs and pressurised compositions. The derivative according to the invention may be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilisers, emulsifiers, buffers, preservatives, sweeteners, flavouring agents, suspending agents, thickening agents, colours, viscosity regulators, stabilisers or osmo-regulators. Suitable examples of liquid vehicles for parenteral administration include water (partially containing additives as above, for example, cellulose derivatives, preferably sodium carboxymethyl cellulose solution), alcohols (including monohydric alcohols and polyhydric alcohols, for example, glycols) and their derivatives, and oils (for example, fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurised compositions can be a halogenated hydrocarbon or other pharmaceutically acceptable propellant.

The process for preparing a pharmaceutical composition of the invention may thus comprise the usual steps that are standard in the art.

According to a fourth aspect of the invention, therefore, there is provided a derivative of the first aspect, or a pharmaceutical composition of the third aspect, for use in therapy. A method of treating a subject with a derivative of the invention, or a pharmaceutical composition comprising the same, is also provided. Any of the different derivatives of IL-22 described or envisaged herein are expressly included in these aspects of the invention.

Terms such as "treating" and "therapy", as used herein, expressly include the treatment, amelioration or prevention of a disease, disorder or condition.

The derivative of IL-22 or pharmaceutical composition comprising the same may be administered directly into a subject to be treated. The derivative or pharmaceutical composition may be administered by any means, including by inhalation, by injection, topically, orally, rectally or ocularly. When administered by inhalation, it may be via the nose or the mouth. Preferably, the derivative or pharmaceutical composition is administered by injection, typically subcutaneously or intravenously. As aforementioned, the lipophilicity of the monoacid derivatives of the invention may also be advantageous in topical administration, oral administration, rectal administration (e.g. suppositories or foams) or pulmonary inhalation. The derivatives therefore have a clear advantage over Fc fusions in their flexibility of administration (e.g. by injection, by inhalation, topical application, rectal or oral administration or ocular delivery) because of their smaller size and higher potency. It will be appreciated that administration, into a subject to be treated, of a derivative of the invention will result in the increased circulation time compared to hIL-22, and that this will aide in treating a disease, disorder or condition. As above, "'treating'" also includes ameliorating and preventing a disease, disorder or condition.

Liquid pharmaceutical compositions, which are sterile solutions or suspensions, can be utilised by, for example, intramuscular, intrathecal, epidural, intraperitoneal and particularly subcutaneous or intravenous injection. The derivative may be prepared as a sterile solid composition that may be dissolved or suspended at the time of administration using sterile water, saline or other appropriate sterile injectable medium.

Forms useful for inhalation include sterile solutions, emulsions and suspensions. Alternatively the derivatives may be administered in the form of a fine powder or aerosol via a Dischaler^{®} or Turbohaler^{®}. Nasal inhalations may suitably be in the form of a fine powder or aerosol nasal spray or modified Dischaler^{®} or Turbohaler^{®}.

Topical formulations include solutions, creams, foams, gels, lotions, ointments, pastes, tinctures and powders. They may be epicutaneous, i.e. applied directly to the skin, or applied to mucous membranes.

Oral administration may be suitably via a tablet, a capsule or a liquid suspension or emulsion. Suitable examples of liquid vehicles for oral administration include water (partially containing additives, for example, cellulose derivatives, preferably sodium carboxymethyl cellulose solution), alcohols (including monohydric alcohols and polyhydric alcohols, for example, glycols) and their derivatives, and oils (for example, fractionated coconut oil and arachis oil). The derivatives of the invention may be administered orally in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like. Solutions, syrups and elixirs also form part of the invention. The derivatives according to the invention can also be administered orally in solid composition form. Solid compositions suitable for oral administration include pills, capsules, granules, tablets and powders.

Rectal administration may suitably be via a suppository or foam.

Formulations for ocular administration are typically solutions, suspensions and ointments for topical application, e.g. in the form of eye drops. Alternatively sterile solutions or suspensions can be utilised by intraocular injection. The derivative may be prepared as a sterile solid composition that may be dissolved or suspended at the time of administration using sterile water, saline or other appropriate sterile injectable medium. The formulation may be for subconjunctival, intravitreal, retrobulbar or intracameral injection.

A derivative or pharmaceutical composition of the invention may be administered to any subject in need thereof. A "subject", as used herein, may be a vertebrate, mammal or domestic animal. Hence, derivatives and compositions according to the invention may be used to treat any mammal, for example livestock (for example, a horse), pets, or may be used in other veterinary applications. Most preferably, the subject is a human being. The derivatives and compositions need not only be administered to those already showing signs of a disease, disorder or condition. Rather, they can be administered to apparently healthy subjects as a purely preventative measure against the possibility of such a disease, disorder or condition in future.

It will be appreciated that derivatives of IL-22 and compositions according to the invention may be used in a monotherapy (i.e. the sole use of that derivative or composition), for treating a disease, disorder or condition. Alternatively, derivatives and compositions according to the invention may be used as an adjunct to, or in combination with, known therapies for treating a disease, disorder or condition.

It will be appreciated that the amount of the derivative of IL-22 that is required is determined by its biological activity, half-life and bioavailability, which in turn depends on the mode of administration, the physiochemical properties of the derivative and composition, and whether it is being used as a monotherapy or in a combined therapy. The frequency of administration will also be influenced by the half-life of the derivative within the subject being treated. Optimal dosages to be administered may be determined by those skilled in the art, and will vary with the particular derivative in use, the strength of the pharmaceutical composition, the mode of administration, and the advancement of the disease, disorder or condition. Additional factors depending on the particular subject being treated will result in a need to adjust dosages, including subject age, weight, gender, diet and time of administration.

Generally, a daily dose of between 0.001 µg/kg of body weight and 10 mg/kg of body weight of derivative of IL-22 according to the invention may be used for treating a disease, disorder or condition, depending upon which derivative or composition is used. More preferably, the daily dose is between 0.01 µg/kg of body weight and 1 mg/kg of body weight, more preferably between 0.1 µg/kg and 500 µg/kg body weight, and most preferably between approximately 0.1 µg/kg and 100 µg/kg body weight.

The derivative of IL-22 or composition may be administered before, during or after onset of the disease, disorder or condition. Daily doses may be given as a single administration (for example, a single daily injection). Alternatively, the derivative or composition may require administration twice or more times during a day. As an example, derivatives may be administered as two (or more depending upon the severity of the disease, disorder or condition being treated) daily doses of between 0.07 µg and 700 mg (i.e. assuming a body weight of 70 kg). A patient receiving treatment may take a first dose upon waking and then a second dose in the evening (if on a two-dose regime) or at 3- or 4-hourly intervals thereafter. Doses may alternatively be given once a week, every fortnight or once a month, or more frequently, for example, two or three times weekly. Known procedures, such as those conventionally employed by the pharmaceutical industry (for example, *in vivo* experimentation, clinical trials, etc.), may be used to form specific formulations of the derivatives and compositions according to the invention and precise therapeutic regimes (such as daily doses of the agents and the frequency of administration).

Many studies have demonstrated key effects of IL-22 in multiple epithelial injury models in especially lung, liver, intestine, kidney, skin, pancreas and thymus. Mechanistically, several pathways within e.g. anti-apoptosis, proliferation, innate immunity, anti-oxidative stress, anti-fibrosis, and stem cell/progenitor cell recruitment have been well documented to meditate IL-22 effects in studies by multiple investigators. Key mechanistic findings have been further confirmed *in vitro* using human cell lines or in human *ex vivo* models (e.g. primary human intestinal organoids). The strong role of IL-22 in preventing cell death, securing regeneration, and controlling inflammation in epithelial injury is therefore well established.

Many studies are performed by analysing genetic models (IL-22 knock-out or transgenic overexpression) subjected to injury. In these studies, the lack of IL-22 or overexpression of IL-22 will be there at the time of injury. In other studies, IL-22 is neutralised with antibodies at the time of injury and, in some cases, IL-22 is neutralised beyond the acute injury phase (e.g. sub-acutely or well into a regeneration phase). Other studies move closer to a treatment scenario by looking at effects of exogenously administered IL-22. Important to note, when looking holistically at the available literature, is that the different models, whether knock-out, overexpression, IL-22 neutralisation before or after injury or exogenous protein dosing, paint the same picture of IL-22 protecting the injured organs and driving regeneration. This indicates a broad application and a broad time window for IL-22 treatment potential, and also shows why a longer-acting IL-22 protein than hIL-22 is required.

Thus, according to a fifth aspect of the invention, there is provided a derivative of the first aspect, or a pharmaceutical composition of the third aspect, for use in a method of treating a metabolic, liver, pulmonary, gut, kidney, CNS or skin disease, disorder or condition. Any of the different derivatives of IL-22 described or envisaged herein are expressly included in this aspect of the invention.

The metabolic disease, disorder or condition may be obesity, diabetes type 1, diabetes type 2, hyperlipidemia, hyperglycemia or hyperinsulinemia.

The liver disease, disorder or condition may be NAFLD, NASH, cirrhosis, alcoholic hepatitis, acute liver failure, chronic liver failure, ACLF, acetaminophen induced liver toxicity, acute liver injury, sclerosing cholangitis, biliary cirrhosis or a pathological condition caused by surgery or transplantation.

The pulmonary disease, disorder or condition may be COPD, cystic fibrosis, bronchiectasis, idiopathic pulmonary fibrosis, acute respiratory distress syndrome, a chemical injury, a viral infection, a bacterial infection or a fungal infection.

The gut disease, disorder or condition may be IBD, ulcerative colitis, Crohn's disease, GvHD, a chemical injury, a viral infection or a bacterial infection.

The kidney disease, disorder or condition may be acute kidney disease or chronic kidney disease.

The CNS disease, disorder or condition may be multiple sclerosis.

The skin disease, disorder or condition may be a wound, inflammatory disease or GvHD

A method of treating a subject having a condition responsive to IL-22 treatment, such as one or more of the above diseases, disorders or conditions, with a derivative of IL-22, or a pharmaceutical composition comprising the same, is also provided.

The derivative of IL-22 has all of the features specified for the first aspect of the invention. The pharmaceutical composition has all of the features specified for the third aspect of the invention. The method of treating a subject having a condition responsive to IL-22 treatment, such as one or more of the above diseases, disorders or conditions, has all of the features specified for the fourth aspect of the invention.

There is no restriction on which derivative of IL-22 or composition as described herein should be administered to which patient. Rather, it is intended that any of the derivatives and compositions described herein can be administered to any patient as described herein.

All of the features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made to the Examples, which are not intended to limit the invention in any way.

### EXAMPLE

The materials and methods employed in the study described in the Example were as follows, unless where otherwise indicated.

### Derivatives & Comparator

Table 4 provides an overview of the derivatives of IL-22 and comparator represented in the data sets.

The derivatives of IL-22 had different backbones and sites of covalent attachment. The linker used in both cases was γGlu-OEG-OEG-C₂DA-Ac. The linker was attached to residue 95C or residue 106C.

**Table 4: Overview of key derivatives and comparator represented in data sets**

| **ID** | **SEQ ID NO. (for IL-22 protein)** | **Backbone variation** | **Fatty acid or other protractor** |
|---|---|---|---|
| hIL-22 | 1 | None | None |
| Derivative 1 | 19 | N35Q, N64Q, L106C substitutions | C18 diacid |
| Derivative 2 | 20 | N35Q, N64Q, R95C substitutions | C18 diacid |

The following is an exemplified protocol, merely intended to illustrate the claimed invention.

### Example 1 - In Vitro Potency Study

### Methods

A reporter gene assay was employed to study potency in BHK cells, which had been triple transfected with IL-22Ra, IL-10Rb and a luciferase with STAT3-induced promoter. This is a highly sensitive, high-throughput assay, which measured IL-22 receptor-mediated STAT3 activation.

A stable reporter BHK cell line was generated using the following plasmids: (i) hIL-10Rb in pcDNA3,1hygro(+), (ii) IL22R in pcDNA3,1(Zeocin) and (iii) 2xKZdel2 in pGL4.20. The cell line hence expressed the human IL-10Rb, human IL-22Ra and luciferase reporter under control of a pSTAT3 driven promoter.

On Day 0 of the assay protocol, the cells were seeded in basal media (for 500 ml: DMEM+Glutamax (Gibco, cat. no.: 31966-021), 10% (w/v) fetal calf serum (FCS; contains albumin) (50 ml) and 1% (w/v) penicillin-streptomycin (P/S) (5 ml)) at 15,000-20,000 cells/well in a 96-well plate (Corning #3842, black, clear bottom). On Day 1, the media was removed by inverting the plate. Fresh basal media was added, at 50 µl per well, and the cells were incubated for 60 minutes.

The derivatives of IL-22 were tested alongside hIL-22 as a comparator in duplicate.

Thus, 50 µl of a diluted derivative or comparator (diluted in basal media) was added to each well and the plate left for four hours. The derivatives and comparators were therefore 2x diluted, as they were diluted into the 50 µl media already in the wells. The stimulation was ended after four hours by adding 100 µl Steadylite plus reagent (Perkin Elmer cat no. 6066759). The plate was sealed with TopSeal A, shaken at 450 rpm for 15 minutes, then read using Mithras or a similar system no later than after 12 hours.

Data analysis was performed using Graphpad Prism. The half maximal effective concentration (EC₅₀) of each derivative or comparator was assessed as a measure of its potency. EC₅₀ was determined using Log(compound) vs response - variable slope (4p). Hill slope was constrained to 1 as standard.

### Results

Table 5 shows the EC₅₀ of the derivatives and comparator measured in the BHK cell reporter gene assay for IL-22 receptor mediated STAT3 activation.

**Table 5: EC₅₀ values for key derivatives and comparator in BHK cell assay**

| **ID** | **ECso (nM)** |
|---|---|
| hIL-22 | 0.07 |
| Derivative 1 | 0.23 |
| Derivative 2 | 0.36 |

As the BHK cell assay contained high amounts of albumin, the measured EC₅₀ incorporated the effect of albumin binding when testing the derivatives.

In the BHK cell assay with albumin present, Derivative 1 had a three-fold reduced potency compared to hIL-22 and Derivative 2 had a five-fold reduced potency compared to hIL-22.

### Conclusion

In the BHK cell assay with albumin in the media, Derivatives 1 and 2 showed only a three- or five-fold reduction in potency compared to hIL-22. In comparison, and as aforementioned, Genentech reports a 34-fold reduction in *in vitro* potency for its Fc fusion of IL-22.

Thus, derivatives of IL-22 are similar in potency with hIL-22 even in the presence of albumin. Cys and Gln substitutions and fatty acid covalent attachment are tolerated in different positions. Indeed, the inventors have shown that conjugation to residues 95 and 106, with the natural amino acids substituted to Cys, has minimal impact on the activity of the IL-22 protein and are therefore considered particularly attractive sites of conjugation. The natural amino acids are R95 and L106.

The data hence illustrate that the derivatives of the invention demonstrate good bioavailability and potency, so offering a new and improved treatment for a diverse range of indications, including metabolic, liver, pulmonary, gut, kidney, CNS and skin diseases, disorders and conditions.

## Claims

1. A derivative of IL-22 comprising a fatty acid covalently attached to an IL-22 protein, wherein the IL-22 protein is a variant of hIL-22, said variant comprising a substitution at position 95 or 106 of hIL-22, and the fatty acid is covalently attached at said substituted position.

2. A derivative as claimed in claim 1, wherein the substitution is selected from R95C and L106C.

3. A derivative as claimed in claim 1, wherein the fatty acid is covalently attached to the IL-22 protein by a linker.

4. A derivative as claimed in any of the preceding claims, wherein the fatty acid is:
(i) of Formula I:
HOOC-(CH₂)ₓ₋CO-*,
wherein x is an integer in the range of 10-18, optionally 12-18, 14-16 or 16-18, and * designates a point of attachment to the IL-22 protein or linker;
(ii) a fatty diacid;
(iii) a C12, C14, C16, C18 or C20 diacid;
(iv) a C16 or C18 diacid;
(v) a C18 diacid;
(vi) of Formula II:
H₃C-(CH₂)ₓ-CO-*,
wherein x is an integer in the range of 10-18, optionally 12-18, 14-16 or 16-18, and * designates a point of attachment to the IL-22 protein or linker;
(vii) a fatty monoacid;
(viii) a C12, C14, C16, C18 or C20 monoacid;
(ix) a C16 or C18 monoacid; and/or
(x) a C16 monoacid.

5. A derivative as claimed in any of the preceding claims, wherein the variant:
(i) is further substituted at position 1, 21, 35, 64, 113 and/or 114 of hIL-22;
(ii) further comprises a substitution of hIL-22 selected from the group consisting of A1C, A1G, A1H, N21C, N21D, N21Q, N35C, N35D, N35H, N35Q, N64C, N64D, N64Q, N64W, Q113C, Q113R, K114C and K114R; and/or
(iii) further comprises a Gln residue at position 35 and 64 of hIL-22.

6. A derivative as claimed in any of the preceding claims, wherein the variant:
(i) is an extended form of hIL-22;
(ii) comprises an N-terminal peptide;
(iii) comprises an N-terminal trimer; and/or
(iv) comprises an N-terminal G-P-G.

7. A derivative as claimed in any of claims 3-6, wherein the linker comprises:
(i) one or more amino acids, optionally including Glu and/or Lys;
(ii) an oxyethylene glycine unit or multiple linked oxyethylene glycine units, optionally 2-5 such units;
(iii) one or more oligo(ethylene glycol) (OEG) residues;
(iv) an ethylenediamine (C₂DA) group;
(v) an acetamide (Ac) group;
(vi) γGlu-OEG-OEG-C₂DA-Ac;
(vii) γGlu-γGlu-γGlu-γGlu-OEG-OEG-εLys-αAc; and/or
(viii) γGlu-OEG-OEG-εLys-αAc

8. A derivative as claimed in any of the preceding claims, wherein the derivative comprises a C18 diacid covalently attached by a linker to the variant of hIL-22.

9. A derivative as claimed in claim 8, wherein the variant comprises a Cys residue substituted at position 95 or 106 of hIL-22 and the linker is attached to said Cys residue.

10. A derivative as claimed in any of the preceding claims, wherein the derivative is Derivative 1 or 2 as identified herein.

11. A process for preparing a derivative as claimed in any of the preceding claims, comprising covalently attaching a fatty acid to an IL-22 protein, wherein the IL-22 protein is a variant of hIL-22, said variant comprising a substitution at position 95 or 106 of hIL-22, and the process comprises covalently attaching the fatty acid at said substituted position.

12. A pharmaceutical composition comprising a derivative as claimed in any of claims 1-10 and a pharmaceutically acceptable vehicle.

13. A derivative as claimed in any of claims 1-10 or a pharmaceutical composition as claimed in claim 12, for use in therapy.

14. A derivative as claimed in any of claims 1-10 or a pharmaceutical composition as claimed in claim 12, for use in a method of treating a metabolic, liver, pulmonary, gut, kidney, central nervous system (CNS) or skin disease, disorder or condition.

15. A derivative or pharmaceutical composition as claimed in claim 14, wherein:
(i) the metabolic disease, disorder or condition is obesity, diabetes type 1, diabetes type 2, hyperlipidemia, hyperglycemia or hyperinsulinemia;
(ii) the liver disease, disorder or condition is non-alcoholic fatty liver disease (NAFLD), non-alcoholic steatohepatitis (NASH), cirrhosis, alcoholic hepatitis, acute liver failure, chronic liver failure, acute-on-chronic liver failure (ACLF), acetaminophen induced liver toxicity, acute liver injury, sclerosing cholangitis, biliary cirrhosis or a pathological condition caused by surgery or transplantation;
(iii) the pulmonary disease, disorder or condition is chronic obstructive pulmonary disease (COPD), cystic fibrosis, bronchiectasis, idiopathic pulmonary fibrosis, acute respiratory distress syndrome, a chemical injury, a viral infection, a bacterial infection or a fungal infection;
(iv) the gut disease, disorder or condition is inflammatory bowel disease (IBD), ulcerative colitis, Crohn's disease, graft-versus-host-disease (GvHD), a chemical injury, a viral infection or a bacterial infection;
(v) the kidney disease, disorder or condition is acute kidney disease or chronic kidney disease;
(vi) the CNS disease, disorder or condition is multiple sclerosis; or
(vii) the skin disease, disorder or condition is a wound, inflammatory disease or GvHD
